# EUROPEAN PATENT SPECIFICATION

(11) **EP 1 947 171 B1**
(45) Date of publication and mention of the grant of the patent: **15.06.2011**
(21) Application number: 08101712.1
(22) Date of filing: 23.06.2004
(51) Int. Cl.: C12N 1/02, C12N 1/20

(54) **Purified bacterial minicells**
Aufgereinigte bakterielle Minizellen
Minicellules bactériennes purifiées

(30) Priority: 24.06.2003 US 602021
(43) Date of publication of application: 23.07.2008
(62) Divisional of application: 04743793.4
(73) Proprietor: EnGeneIC Molecular Delivery Pty Ltd., North Ryde, Sydney NSW 2113 (AU)
(72) Inventor: Brahmbhatt, Himanshu, Sydney, New South Wales 2176 (AU); MacDiarmid, Jennifer, Sydney, New South Wales 2037 (AU)
(74) Representative: Plougmann & Vingtoft A/S

(56) References cited:
- WO-A-03/033519
- KHACHATOURIANS G G ET AL: "A NEW METHOD FOR THE PREPARATION OF MINICELLS FOR PHYSIOLOGICAL STUDIES" PREPARATIVE BIOCHEMISTRY, NEW YORK, NY, US, vol. 3, no. 3, 1 January 1973 (1973-01-01), pages 291-298, XP009066765
- KHACHATOURIANS G G ET AL: "Expression of recombinant DNA functonal products in Escherichia coli anucleate minicells" BIOTECHNOLOGY ADVANCES, ELSEVIER PUBLISHING, BARKING, GB, vol. 4, no. 1, 1 January 1986 (1986-01-01), pages 75-93, XP002381588 ISSN: 0734-9750
- STIEGLITZ H ET AL: "CLONING, SEQUENCING, AND EXPRESSION IN FICOLL-GENERATED MINICELLS OF AN ESCHERICHIA COLI HEAT-STABLE ENTEROTOXIN GENE" PLASMID, NEW YORK,NY, US, vol. 20, no. 1, 1 July 1988 (1988-07-01), pages 42-53, XP009066759 ISSN: 0147-619X
- CHRISTEN A C ET AL: "Rapid isolation of escherichia coli minicells by glass-fiber filtration: a study of plasmid-coded polypeptides" GENE, ELSEVIER, AMSTERDAM, NL, vol. 23, no. 2, 1 August 1983 (1983-08-01), pages 195-198, XP003000864 ISSN: 0378-1119

## Description

### BACKGROUND OF THE INVENTION

The present invention relates to purified bacterial minicells.

A minicell is an anucleate form of an *E. coli* or other bacterial cell, engendered by a disturbance in the coordination, during binary fission, of cell division with DNA segregation. Prokaryotic chromosomal replication is linked to normal binary fission, which involves mid-cell septum formation. In *E*. *coli,* for example, mutation of *min* genes, such as *min*CD, can remove the inhibition of septum formation at the cell poles during cell division, resulting in production of a normal daughter cell and an anulceate minicell (de Boer *et al.,* 1992; Raskin & de Boer, 1999; Hu & Lutkenhaus, 1999; Harry, 2001).

In addition to *min* operon mutations, anucleate minicells also are generated following a range of other genetic rearrangements or mutations that affect septum formation, for example in the *div*IVB1 in *B. subtilis* (Reeve and Cornett, 1975; Levin *et al.,* 1992). Minicells also can be formed following a perturbation in the levels of gene expression of proteins involved in cell division/chromosome segregation. For example, overexpression of *min*E leads to polar division and production of minicells. Similarly, chromosome-less minicells may result from defects in chromosome segregation, for example the *smc* mutation in *Bacillus subtilis* (Britton *et al.,* 1998), *spo*OJ deletion in *B. subtilis* (Ireton *et al.,* 1994), *muk*B mutation in *E*. *coli* (Hiraga *et al.,* 1989), and *par*C mutation in *E*. *coli* (Stewart and D'Ari, 1992). Gene products may be supplied *in trans.* When over-expressed from a high-copy number plasmid, for example, CafA may enhance the rate of cell division and/or inhibit chromosome partitioning after replication (Okada *et al.,* 1994), resulting in formation of chained cells and anucleate minicells (Wachi *et al.,* 1989; Okada *et al.,* 1993).

Minicells are distinct from other small vesicles that are generated and released spontaneously in certain situations and, in contrast to minicells, are not due to specific genetic rearrangements or episomal gene expression. Exemplary of such other vesicles are bacterial blebs, which are small membrane vesicles (Dorward *et al.,* 1989). Blebs have been observed in several bacterial species from *Agrobacterium, Bacillus, Bordetella, Escherichia, Neisseria, Pseudomonas, Salmonella* and *Shigella,* for example. Bacterial blebs can be produced, for instance, through manipulation of the growth environment (Katsui *et al*., 1982) and through the use of exogenous membrane-destabilizing agents (Matsuzaki *et al.,* 1997).

Because plasmid replication within prokaryotic cells is independent of chromosomal replication, plasmids can segregate into both normal daughter cells and minicells during the aberrant cell division described above. Thus, minicells derived from recombinant *min E. coli* carry significant numbers of plasmid copies, with all of the bacterial cellular components except for chromosomes, and have been used as such in studying plasmid-encoded gene expression *in vitro.* See Brahmbhatt (1987), Harlow *et al.* (1995), and Kihara *et al.* (1996). Brahmbhatt (1987) demonstrated, for example, that *E. coli* minicells can carry recombinant plasmids with DNA inserts as large as 20 kb, absent any chromosomal DNA, and can express nine or more recombinant proteins simultaneously.

A recent patent application, WO 03033519, described recombinant, intact minicells containing therapeutic nucleic acid molecules. Such minicells are effective vectors for delivering oligonucleotides and polynucleotides to host cells *in vitro* and *in vivo.* Accordingly, they are particularly useful for introducing nucleic acid molecules that, upon transcription and/or translation, function to ameliorate or otherwise treat a disease or modify a trait associated with a particular cell type, tissue or organ of a subject.

*In vivo* minicell applications generally require minicell preparations of a high purity, particularly with respect to live parent bacteria, free endotoxin and cellular debris (including dead parent bacteria, membrane fragments, nucleic acids and intracellular components) that might elicit an inflammatory response in an immunized host. Moreover, the use of minicells in commercial pharmaceutical products will require methods for purifying minicells to approved international pharmaceutical standards. To this end, conventional methods of minicell purification generally are unsatisfactory.

Conventional techniques entail (a) low speed centrifugation, to reduce the bio-burden of parent cells, and (b) differential rate sedimentation in a gradient of glycerol, sucrose or percoll. An initial differential, low speed centrifugation typically reduces parental cells by as much as 100-fold, while leaving 50% to 70% of minicells in the supernatant fluid. Two subsequent cycles of differential rate sedimentation then yield minicell preparations having a purity of about 1 vegetative cell per 10⁶ - 10⁷ minicells. Such conventional methods are reviewed by Frazer & Curtiss (1975), and are described by Reeve (1979), Clark-Curtiss & Curtiss (1983), and U.S. Patent No. 4,311,797 (to Khachatourians).

The purity achieved by conventional purification methods may not be adequate for all *in vivo* applications, some of which may require doses greater than 10⁶ minicells, or even 10¹⁰ minicells. At the aforementioned contamination ratio, this would translate into 10,000 live parent cells per dose. Such a contamination level could be fatal, particularly in immuno-compromised patients such as cancer and AIDS patients. For example, the ID₅₀ (infectious dose in 50% of infected people) for *Shigella dysenteriae, Salmonella enteritidis* and *Listeria monocytogenes* organisms is approximately 10, 1,000 and 10 respectively. Moreover, previous studies have reported that the level of parental cell contamination varies with different bacterial strains (Clarke-Curtiss and Curtiss, 1983). In that regard, gene therapy applications described in WO 03033519 may employ minicells derived from a range of mutant Gram-negative and Gram-positive bacterial strains, and would require minicells that are essentially free of live parent bacterial cell contamination. Thus, conventional minicell purification methods do not permit quality control for cGMP (current good manufacturing practice) manufacture of biopharmaceutical doses of minicells.

As an additional drawback, the gradient formation media (percoll, sucrose and glycerol) employed by conventional purification methods are incompatible with *in vivo* uses. Percoll is toxic and, hence, is restricted to "research purposes only" contexts. Sucrose imparts a high osmolarity to gradients that can cause physiological changes in minicells. Indeed, the present inventors have determined that minicells undergo an osmotic shock in sucrose gradients and, as a consequence, become structurally deformed. Glycerol is highly viscous and difficult to remove completely from the minicell suspensions. Accordingly, although these density gradient media effectively separate cells and cellular organelles or components, they are not suitable for separating biological cells that are destined for clinical use in humans.

Several approaches have been developed to improve conventional minicell purification techniques. One approach employs parent cells that carry a chromosomal *rec*A mutation, and treatment with low doses of Ultra Violet (UV) radiation (Sancar *et al.,* 1979). The rationale of this approach is that UV radiation will preferentially degrade chromosomal DNA because of its large target size, as opposed to smaller plasmid DNA. However, recombinant minicells used for gene therapy and vaccine applications must be free of any mutation, and non-specific mutagenesis methods such as UV radiation would not ensure that all plasmid DNAs remain un-mutated.

Another approach to improve minicell purification operates by inhibiting bacterial cell wall synthesis, such as by using ampicillin or cycloserine, or by starving diaminopimelic acid (DAP)-requiring strains of DAP (Clarke-Curtiss and Curtiss, 1983). This approach also suffers from several drawbacks, however. First, many recombinant plasmids used for gene therapy will carry an ampicillin resistance marker, which renders parent cells carrying the plasmid ampicillin resistant. Second, many *in-vivo* minicell applications will employ minicells derived from a range of different bacterial species, many of which may not be susceptible to DAP-requiring mutations. Third, any large-scale use of antibiotics is undesirable due to the attendant risks of generating antibiotic-resistant bacteria.

Recently, a novel approach for purifying minicells that addresses the above-mentioned concerns was reported (WO 03033519). The novel method combines cross-flow filtration (feed flow is parallel to a membrane surface; Forbes, 1987) and dead-end filtration (feed flow is perpendicular to the membrane surface) to achieve a minicell purity that exceeds 10⁻⁷ (*i.e*., fewer than one parent cell per 10⁷ minicells), and even 10⁻⁹. Optionally, the filtration combination can be preceded by a differential centrifugation, at low centrifugal force, to remove some portion of the bacterial cells and thereby enrich the supernatant for minicells.

Although this filtration procedure overcomes the drawbacks associated with conventional minicell purification techniques, it also has limitations. Foremost, cross-flow filtration results in considerable loss of minicells, which adds cost to the manufacturing process. Additionally, minicell preparations obtained by the filtration procedure contain some bacterial endotoxin, which causes a mild shock when administered *in vivo.* Finally, minicell purity varies from batch to batch when the filtration methods are employed.

A need remains for high purity bacterial minicells.

### SUMMARY OF THE INVENTION

The present invention provides purified minicell preparations, prepared according to the methods disclosed below. The purified minicell preparations contain fewer than about 1 contaminating parent bacterial cell per 10¹⁰ or 10¹¹ minicells. Also preferably, the purified minicell preparations are substantially free from endotoxin and cellular debris (including dead parent bacteria, membrane fragments, nucleic acids and intracellular components) that might elicit an inflammatory response or endotoxic shock in an immunized host.

### BRIEF DESCRIPTION OF THE DRAWINGS

**Figure 1** depicts one way in which minicell purification techniques of the present invention can be integrated with other minicell purification procedures.
**Figure 2** shows Scanning Electron Micrographs of *S*. *typhimurium min*CDE-strain bacteria (range of different sizes) and minicells derived from the strain. **(A)** shows a small sized parent bacterium (1.1 um long) and a minicell (0.4 um diameter), **(B)** shows a larger parent bacterium (1.32 um long), **(C)** shows an even larger parent bacterium (1.6 um long), and **(D)** shows a mixture of parent bacteria and minicells, where the former range in length from 1 um to 4 um.
**Figure 3A** shows the filamentation of parent *S*. *typhimurium* minCDE-strain bacteria following incubation with various NaCl concentrations for various times.
**Figure 3B** shows fluorescence microscope images comparing the sizes of *S*. *typhimurium min*CDE-strain bacteria incubated in growth media for 4 hrs in the absence of NaCl (left side image) and of bacterial filaments formed after 4 hrs incubation in the presence of 5 % NaCl (right side image).
**Figure 4A** shows the filamentation of parent *E*. *coli min*CDE-strain bacteria following incubation with various NaCl concentrations for various times.
**Figure 4B** shows fluorescence microscope images comparing the sizes of *E*. *coli min*CDE-strain bacteria incubated in growth media for 4 hrs in the absence of NaCl (left side image) and of bacterial filaments formed after 4 hrs incubation in the presence of 5% NaCl (right side image).

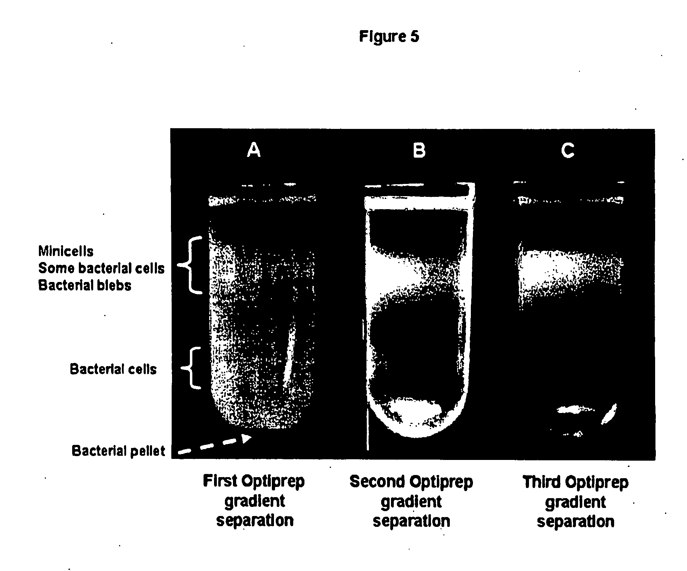

**Figures 5A-C** show 3 sequential purification stages of minicells using biologically compatible media, *e.g*. Optiprep, for density gradient centrifugation as described in Example 3. The crude minicell preparation separated by the first density gradient centrifugation (Fig. 5A) reveals the contaminants, *i.e*., significant bacterial pellet and relatively small sized bacterial cells forming a band between the minicells and the bacterial pellet. The minicell band was collected and processed through a second Optiprep gradient (Fig. 5B) revealing a clearer minicell band, sharper band of small bacterial cells and a negligible bacterial pellet. The minicell band was collected and processed through a third Optiprep gradient (Fig. 5C) revealing a significant purification of the minicells.
**Figures 6A-C** show the counts of minicells (Fig. 6A), viable bacterial cells (Fig. 6B) and Endotoxin (EU; Fig. 6C), respectively, during the steps of a minicell purification procedure. Different stages in the purification process where samples were collected for analysis are shown on the x-axis. Experimental details are described in Example 5. Each value is a mean of three samples and each sample was collected from a separate purification process. Standard error bars are shown.

### DETAILED DESCRIPTION OF PREFERRED EMBODIMENTS

The present inventors have determined that the use of biologically compatible media improves conventional minicell purification. In this regard, they have observed that commonly used density gradient media, while effective at separating minicells from contaminants, often have adverse effects on minicells. For example, conventional methods commonly employ 30% sucrose gradients and require two to three repeated sucrose gradient purifications to achieve adequate purity. This exposes minicells to high osmotic pressure for up to two hours, likely causing osmotic shock to the minicells. The present inventors have found that sucrose-gradient purified minicells often are significantly deformed relative to minicells purified by other means. Presumably, the deformity results from membrane destabilization, which allows excess fluid into the minicells. Such membrane destabilization, and its attendant increase in membrane porosity, also could allow cytosol contents, including therapeutic nucleic acids to leak out of the minicells.

In one aspect, therefore, the present invention contemplates a minicell purification method that comprises separating minicells from parent bacterial cells and other contaminants via density gradient centrifugation in a biologically compatible medium. After centrifugation, a minicell band is collected from the gradient, and, optionally, the minicells may be subjected to further rounds of density gradient centrifugation to maximize purity. The method may further include a preliminary step of performing differential centrifugation on the minicell-containing sample. When performed at low centrifugal force, differential centrifugation will remove some portion of parent bacterial cells, thereby enriching the supernatant for minicells.

"Biologically compatible media," used in this context, refers to media that do not adversely affect minicell physiology or morphology. Preferably, biologically compatible media also do not adversely affect host cell physiology, or host organism physiology. The meaning of "biologically compatible" is therefore contextual. For example, a particular medium may be biologically compatible to one type of minicell, but toxic to another. Preferably biologically compatible media are both isotonic and non-toxic.

OptiPrep™ (Axis-Shield PLC, Dundee, Scotland), which is a sterile 60% (w/v) solution of iodixanol (5,5'-[(2-hydroxy-1-3propanediyl)-bis(acetylamino)] bis [N,N'-bis(2,3dihydroxypropyl-2,4,6-triiodo-1,3-benzenecarboxamide]) in water, constitutes one highly preferred example of a biologically compatible medium. Researchers have extensively utilized OptiPrep™ and other similar density gradient media for purifying mammalian cells and organelles, as well as membrane vesicles, viruses, proteins, nucleic acids and lipoproteins. These uses are reviewed in *Density Gradient Media. Applications and Products 2002,* Axis-Shield PLC, Dundee, Scotland. Such media were not previously employed, however, to purify bacterially-derived minicells. Indeed, prior to the present inventors' observation that other media adversely affect minicell physiology and morphology, a need for biologically compatible media to purify minicells was not even recognized.

With OptiPrep™ it is possible to use either preformed gradients, or to form a gradient in situ by centrifugation (self-generating gradient). Preformed gradients can be continuous or discontinuous gradients. Preformed gradients of OptiPrep™ can be formed by layering solutions of the desired concentrations into a centrifuge tube and allowing the solutions to diffuse by sealing the top of the tube and laying it on its side during diffusion. The preparation of isoosmotic density gradients with OptiPrep™ depends upon preparing gradient solutions by dilution of OptiPrep™ solution with an appropriate diluent solution. Selection of a diluent solution and osmotic balancers is well within the ordinary skill or practitioners.

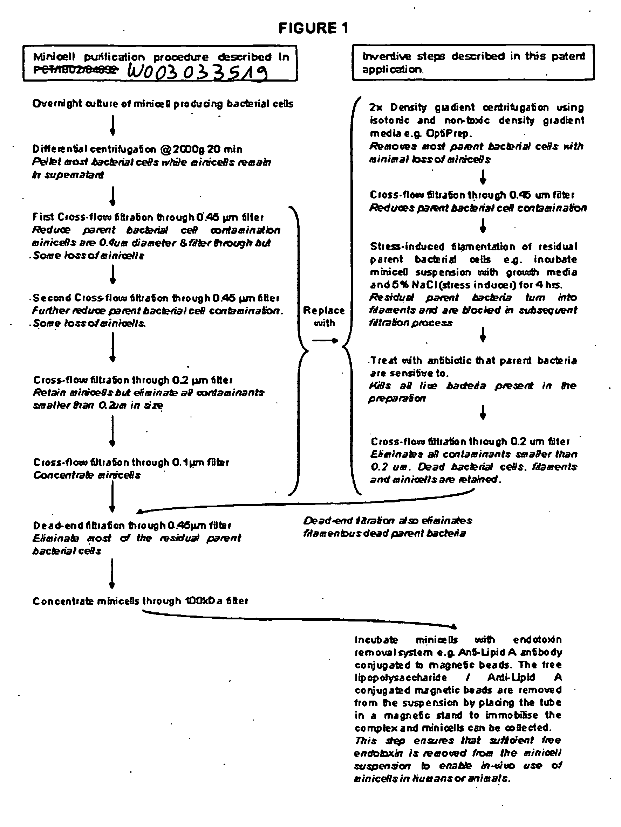

In another aspect, the present invention combines density gradient centrifugation in a biologically compatible medium with filtering steps. For example, density gradient centrifugation can be incorporated into a serial filtration process, as exemplified in Figure 1. One such serial filtration process is described in WO 03033519. Briefly, that process combines cross-flow filtration (feed flow is parallel to a membrane surface) and dead-end filtration (feed flow is perpendicular to a membrane swface). Optionally, this combination can be preceded by a differential centrifugation, at low centrifugal force, to remove some portion of parent bacterial cells and thereby enrich the supernatant for minicells. Also optionally, the combination can be followed by an antibiotic treatment to kill residual parent bacterial cells.

Cross-flow filtration, depending on the filter pore size, can separate minicells from larger contaminants such as parent bacterial cells, and from smaller contaminants such as bacterial blebs, free endotoxin, nucleic acids, cellular debris and excess liquid. To separate minicells from larger contaminants, the nominal pore size of cross-flow filters should allow minicells to permeate through the filters, but not large bacterial cells. A 0.45 µm pore size is preferred for this purpose because minicells are approximately 0.4 µm in diameter, whilst bacterial cells are larger. To separate minicells from smaller contaminants, the nominal pore size of cross-flow filters should allow smaller contaminants to permeate through the filters, but not minicells. A 0.2 µm pore size is preferred for this purpose because bacterial blebs range in diameter from 0.05 µm to 0.2 µm, and the other smaller contaminants are less than 0.2 µm.

Effective application of cross-flow filtration in this context typically entails at least one step involving a larger pore size, around 0.45 µm, followed by at least one step with a smaller pore size, around 0.2 µm. Between or during serial cross-flow filtration steps, diafiltration may be performed to maximize recovery of minicells. In the diafiltration, volume is held constant and ultrafiltration membranes are used to retain desired particles (minicells, in this case), while undesirable smaller solutes and particles are removed.

The use of cross-flow filtration accommodates suspensions carrying heavy loads of particulate matter, such as bacterial cultures, which may carry loads of 10¹¹ to 10¹³ bacterial and minicell populations per liter of culture. To minimize filter fouling and the consequent loss of minicells, the bacterial/minicell culture may be diluted, preferably 5-fold to 10-fold. Dilutions also permit use of appropriately low pump pressure and flow rate.

To remove residual parent bacterial cells remaining after cross-flow filtration, dead-end filtration may be performed. For this purpose, the use of at least one dead-end filtration, employing a pore size of about 0.45 µm, is preferred.

In one embodiment, a minicell purification method combines density gradient centrifugation through a biologically compatible medium with a filtration step that employs at least one filter with a pore size less than or equal to about 0.2 µm.

In another embodiment, a minicell purification method combines density gradient centrifugation through a biologically compatible medium with a dead-end filtration step employing a filter with a pore size of about 0.45 µm.

The present inventors also have discovered that inducing parent bacterial cells to adopt a filamentous form, prior to filtration, significantly improves minicell purification. Because minicells and parent bacterial cells have the same diameter (average of 0.4 um) some bacterial cells can permeate a filter pore that barely accommodates a minicell (e.g., 0.45 µm cross-flow or dead-end filter pores), even though the length of bacterial cells is at least 1 µm. This occurs when an oblong bacterial cell lodges itself perpendicular to a filter. However, bacterial cell filaments, consisting of bacterial cells joined end-to-end cannot penetrate such filters.

Thus, another aspect of the invention entails inducing contaminating parent bacterial cells to form filaments prior to filtration. This is accomplished by subjecting a minicell suspension to environmental conditions that induce a stress response in parent cells. Such conditions are well known to those skilled in the art, and include anaerobic conditions, nutrient limiting conditions and abnormal osmotic conditions. Hypertonic media are particularly useful for inducing filamentation. In one example, a minicell suspension can be supplemented with Trypticase Soy Broth (growth medium) that contains 5% sodium chloride (stress inducer). Under such stress-inducing conditions, cells fail to fully septate during cell division, and form long bacterial filaments consisting of multiple cells.

Preferred embodiments of the invention exploit bacterial filamentation to increase minicell purity. Thus, in one aspect, the invention provides a minicell purification method that includes the steps of (a) subjecting a sample containing minicells to density gradient centrifugation in a biologically compatible medium, and (b) subjecting the sample containing minicells to a condition that induces parent bacterial cells to adopt a filamentous form, followed by (c) filtering the sample to obtain a purified minicell preparation.

The present inventors have further discovered that the removal of endotoxin improves minicell preparations. In *in vivo* mouse studies, they observed a mild shock resulting from the use of minicell preparations containing residual endotoxins. Thus, useful minicell preparations preferably are substantially free from endotoxins, meaning that they contain clinically insignificant levels of endotoxin, or levels that would not induce an inflammatory response or endotoxic shock in a patient.

Methods for removing endotoxins are well-known in the art. One exemplary method utilizes magnetic beads (for example, Dynabeads™; Dynal biotech, Oslo, Norway) coated with anti-Lipid A antibodies. Antibody coated magnetic beads can be mixed with a minicell suspension in a tube, and incubated to allow antibody to bind to free lipopolysaccharide (LPS) via its Lipid A portion. The tube carrying the suspension is then placed in a magnetic stand to immobilize the anti-Lipid A-LPS complexed magnetic beads, and the minicells are collected. Multiple cycles of incubation with fresh beads can be performed to achieve the desired level of purity.

Monoclonal antibodies that bind to epitopes found in the deep-core polysaccharide part of LPS also are useful for removing free endotoxin. The deep-core polysaccharide part of LPS is not thought to be exposed on bacterial membrane surfaces. Therefore, antibodies directed against this part of LPS should not bind to bacterial cell-bound LPS. Prior to use, such antibodies should be tested to ensure that they do not cross-react with cell-surface exposed components of LPS.

Due to the potential for bacterial endotoxins to cause adverse side effects, preferred minicell purification methods include one or more steps to remove them. Thus, in one aspect, the invention provides a minicell purification method that employs a density gradient centrifugation step in a biologically compatible medium, followed by one or more steps to remove endotoxin from the resulting enriched minicell preparation. More preferably, the method further includes one or more filtration steps, as described above.

The minicell purification techniques described herein may be employed in various combinations to obtain a preparation of a desired purity. Preferred methods include a combination of density gradient centrifugation and filtration. Preferred methods also include stress-induced filamentation of parent bacterial cells followed by filtration, and removal of endotoxin from minicell preparations. One example of a method (schematically depicted in Figure 1) that employs all of these techniques is as follows:
- Step A:: Differential centrifugation of a minicell producing bacterial cell culture. This step, which may be performed at 2000g for about 20 minutes, removes most parent bacterial cells, while leaving minicells in the supernatant.
- Step B:: Density gradient centrifugation using an isotonic and non-toxic density gradient medium. This step separates minicells from many contaminants, including parent bacterial cells, with minimal loss of minicells. Preferably, this step is repeated within a purification method.
- Step C:: Cross-flow filtration through a 0.45 µm filter to further reduce parent bacterial cell contamination.
- Step D:: Stress-induced filamentation of residual parent bacterial cells. This may be accomplished by subjecting the minicell suspension to any of several stress-inducing environmental conditions.
- Step E:: Antibiotic treatment to kill parent bacterial cells.
- Step F:: Cross-flow filtration to remove small contaminants, such as membrane blebs, membrane fragments, bacterial debris, nucleic acids, media components and so forth, and to concentrate the minicells. A 0.2 µm filter may be employed to separate minicells from small contaminants, and a 0.1 µm filter may be employed to concentrate minicells.
- Step G:: Dead-end filtration to eliminate filamentous dead bacterial cells. A 0.45 um filter may be employed for this step.
- Step H:: Removal of endotoxin from the minicell preparation. Anti- Lipid A coated magnetic beads may be employed for this step.

Those skilled in the art can implement variations of these steps and incorporate additional purification steps, consistent with the principles outlined herein.

The foregoing methods for purifying bacterial minicells provide purified minicell preparations useful for *in vivo* applications such as those described in WO 03033519. These preparations contain fewer than about 1 contaminating parent bacterial cell per 10⁷ minicells, preferably fewer than about 1 contaminating parent bacterial cell per 10⁸ minicells, more preferably fewer than about 1 contaminating parent bacterial cell per 10⁹ minicells, even more preferably fewer than about 1 contaminating parent bacterial cell per 10¹⁰ minicells, and yet more preferably fewer than about 1 contaminating parent bacterial cell per 10¹¹ minicells. Preferably, any contaminating parent bacterial cells are dead, and these preparations do not contain any live parent bacterial cells. Also preferably, the purified minicell preparations are substantially free from endotoxin and cellular debris (including dead parent bacteria, membrane fragments, nucleic acids and intracellular components). As previously explained, a minicell preparation is substantially free from endotoxin if the preparation contains clinically insignificant levels of endotoxin, or levels that would not induce an inflammatory response or endotoxic shock in a patient. Similarly, a minicell preparation is substantially free from cellular debris if it contains clinically insignificant levels of cellular debris, or levels that would not induce an inflammatory response in a patient.

Reference to the following, illustrative examples will help to provide a more complete understanding of the invention.

### Example 1 - Inconsistency of filtration without the inventive techniques

This example illustrates that the use of filtration to purify minicells, without the inventive techniques, can produce inconsistent results.

Minicell-producing mutant bacterial strains of *S. typhimurium, E. coli* and *Shigella flexneri* are analyzed by Scanning Electron Microscopy (SEM) to determine the size of the bacterial cells and minicells. For High Resolution Scanning Electron Microscopy the following method is followed. Bacterial cultures are grown in Trypticase Soy Broth (TSB) (BBL brand purchased from Bacto Labs, Liverpool, NSW, Australia). The broth is prepared according to the manufacturer's instructions at 30 gm/l, and autoclaved at 121°C for 15 minutes. Liquid culture is grown overnight in a shaking incubator at 37°C. To change solutions the cells are centrifuged at 13,000 rpm for 20 minutes, the supernatant is discarded, and the cells are resuspended in the new reagent (described below) using a vortex mixer. This washes ions and biomaterials off the cells and leaves them suspended in a small volume of distilled water. The sequence of reagents is (a) 1 ml of distilled water - repellet, (b) 1 ml of distilled water - resuspend, (c) deposit 250 µl on a clean brass specimen plate, (d) dry overnight at 30°C, (e) coat just before microscopy with 2 nm of chromium metal deposited in a Xenosput clean vacuum sputter coater. The coated specimens are examined using an Hitachi S-900 Field Emission Scanning Electron microscope using a beam energy of 3 kilovolts (University of New South Wales, NSW, Australia). Digital images at different magnifications are recorded using an ImageSlave digitizer.

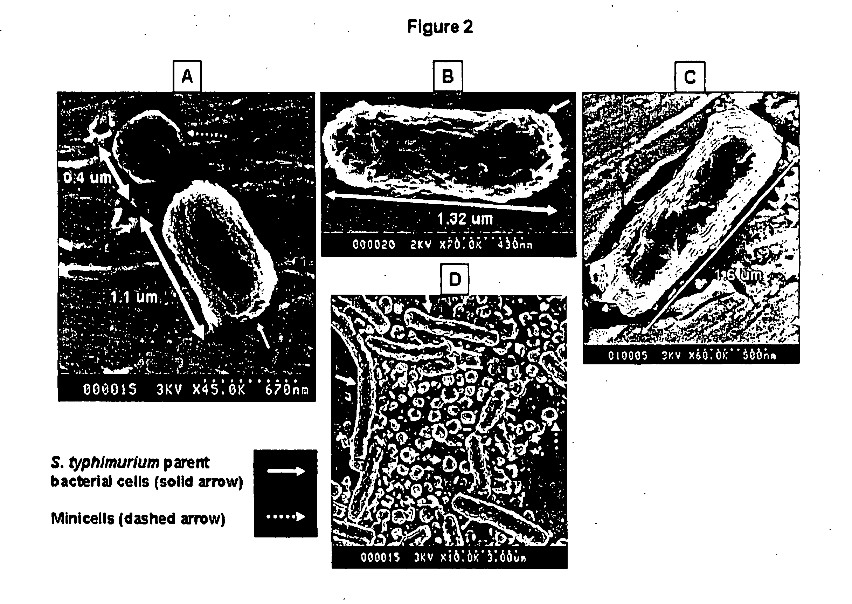

The results show (representative images of *S. typhimurium min*CDE- strain are shown in Figures 2A-D) that parent bacterial cells range in length from 0.9 um to 4 µm and 0.4 µm to 0.5 µm in width. Following filtration steps outlined on the left side of Figure 1, some batches show residual bacterial contamination. The contaminating bacteria are small in size, *i.e*., about 0.9 um in length. This indicates that some small sized bacteria that are approximately the same width as minicells (Fig. 2A) leak through the 0.45 µm, cross-flow and dead-end filters.

### Example 2 - Elimination of small bacteria: conversion into bacterial filaments.

This Example demonstrates that inducing bacteria to filament prior to filtration improves minicell purification processes.

A study is designed to address the problem described in Example 1, by making the residual small-sized parent bacteria substantially larger than the 0.45 µm pore size of a dead-end filter. Stress-inducing conditions in a bacterial growth environment can prevent complete septation during bacterial cell division, resulting in bacterial filaments.

The study demonstrates that hypertonic bacterial growth media (stress-inducer) reliably induce filamentation of minicell-producing bacterial strains of *S*. *typhimurium* and *E. coli.* All bacteria are grown from glycerol stocks maintained at -80°C. *S*. *typhimurium* and *E. coli* strains are grown in Trypticase Soy Broth (TSB) (BBL brand purchased from Bacto Labs, Liverpool, NSW, Australia). It is prepared according to the manufacturer's instructions at 30 gm/l, and autoclaved at 121°C for 15 minutes. Liquid culture is grown in a shaking incubator at 37°C. Overnight bacterial culture is diluted 1:5,000 in fresh TSB and grown until OD₆₀₀ₙₘ reaches 0.2. The culture is divided into ten 5 ml aliquots in sterile vials, and pre-autoclaved sterile NaCl is added to each vial to yield final NaCl concentrations (w/v) of 0% (control), 2 % , 3 % , 4.5 %, 5 %, 5.5 % , 6%, 7 % and 8 %. The cultures are incubated statically at 37°C and samples are obtained at 2 hrs, 4 hrs, 8 hrs and 24 hrs. A zero hour control sample is also obtained for microscopy. The samples are centrifuged at 13,200 rpm and the bacterial / minicell pellets are resuspended in distilled water. A drop of each sample is placed on a glass slide, air dried and heat fixed. Each sample is Gram-stained using a 95 % alcohol wash followed by Gram Safranin flood for 1 min. and a water wash. The slides are visualized using the Leica Model DMLB light microscope with image analysis by means of a Leica DC camera and Leica IM image management software. Samples are viewed at 40X or oil immersion at 100X magnification.

The above-experiments are repeated four times to determine reliability of results and variations with a series of controls also are performed.

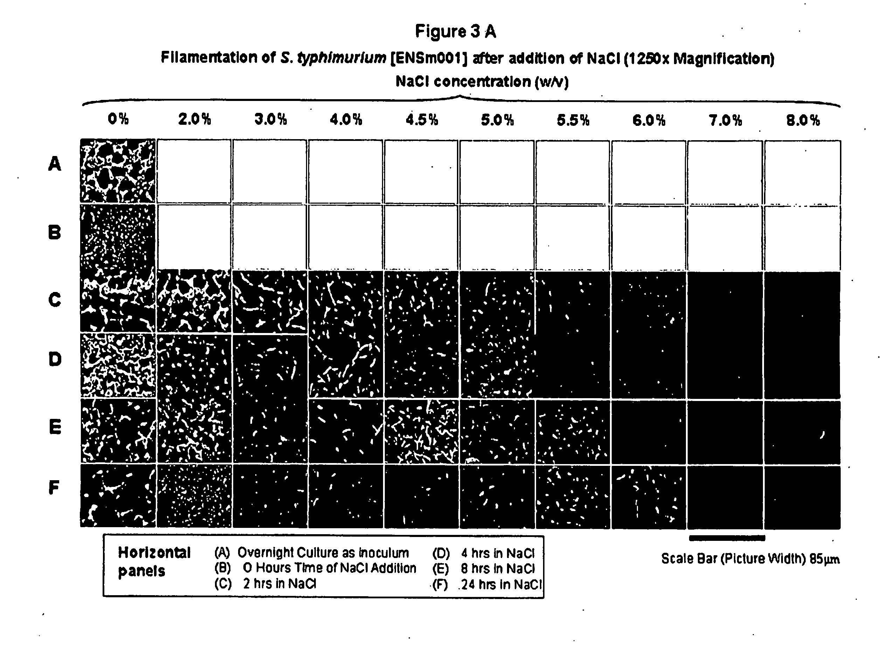

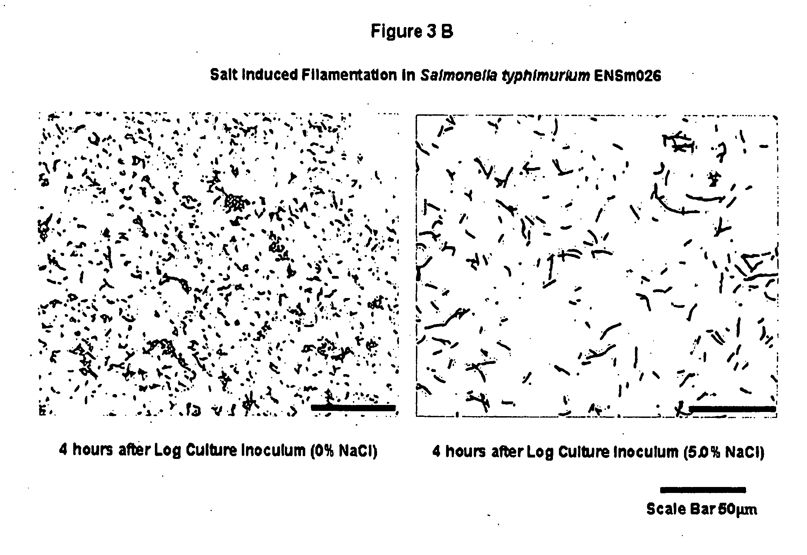

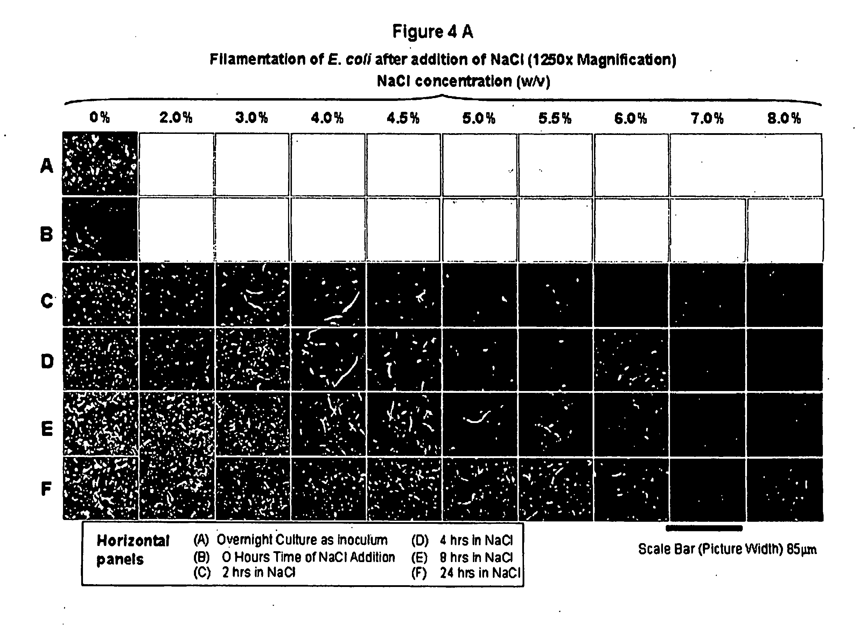

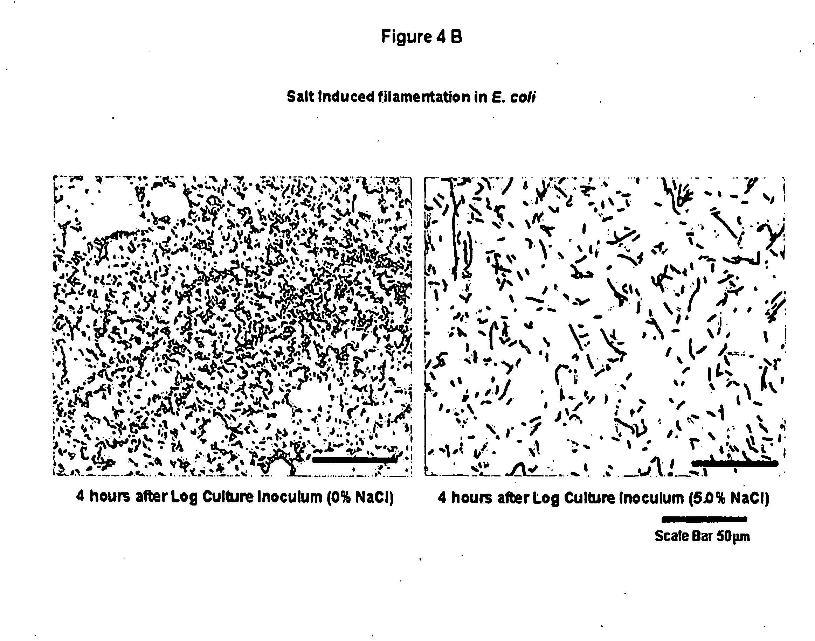

The results show (Figs 3A-B and 4A-B) that with increasing NaCl concentration, the bacterial cells form filaments comprising two to twenty coccobacilli stuck end-to-end. Within the range of 2 % to 3 % NaCl concentrations, filamentation is variable (Figs 3A and 4A), because several bacterial cells do not form filaments even after longer incubation periods. However, at 4 % to 5 % NaCl, the bacterial cells reliably turn into filaments (Figs 3B and 4B). The optimum incubation period for filamentation at 4% to 5% NaCl is about 4 hrs, and further incubation up to 24 hrs is not generally necessary. Higher salt concentrations of 5.5% to 8% decrease filament formation. Preliminary studies to determine a viable bacterial count of each sample by dilution plating on TSB agar plates suggests that significant numbers of bacterial cells are killed at higher salt concentrations (5.5% to 8% NaCl), a potential reason why decreased filamentation is observed at these NaCl concentrations.

A definitive study of the effect of the various NaCl concentrations on the bacterial cell viability is performed out using the LIVE/DEAD BacLight Bacterial Viability Kit (Molecular Probes, Eugene, OR, USA). The kit employs two nucleic acid stains, the green-fluorescent SYTO® 9 stain and the red-fluorescent propidium iodide stain. These stains differ in their ability to penetrate healthy bacterial cells. SYTO 9 stain labels both live and dead bacteria. In contrast, propidium iodide (PI) penetrates only bacteria with damaged membranes, reducing SYTO 9 fluorescence when both dyes are present. Thus, live bacteria with intact membranes fluoresce green, while dead bacteria with damaged membranes fluoresce red. The above-described experiment on salt-induced filamentation is repeated, and 0 hr, 2 hr, 4 hr, 8 hr and 24 hr samples for the various NaCl concentrations are obtained. The samples are centrifuged at 13,200 rpm, supernatant discarded and bacterial/minicell pellet is resuspended in 100 µl of BSG. 0.5 µl of a 50/50 mix of SYTO 9/PI is added to each sample and incubated for 15 min. The samples are centrifuged at 13,200 rpm, supernatant discarded and pellets are resuspended in 100 µl of distilled water. A drop of each sample is placed on a glass slide, air dried and covered with a drop of BacLight Mounting Oil. Each sample is visualized using the Leica Model DMLB light microscope with image analysis by means of a Leica DC camera and Leica digital image acquisition software. Samples are viewed at 40X or oil immersion at 100X magnification.

The results show (color photos not shown) that at NaCl concentrations of 5.5 % and higher, significant numbers of bacterial cells fluoresce red (dead cells) and at NaCl concentrations of 7 % and 8 %, almost all of the bacterial cells are dead within 2 hrs of incubation. This result shows that 4 % to 5 % NaCl for an incubation time of 4 hrs is the maximum limit to achieve filamentation. After 2 hrs of incubation, the live bacterial cells turn into filaments. However as the incubation time increases the filaments fluoresce red, suggesting that even 4 % to 5 % NaCl is sufficient stress for the bacterial cells and that they begin to die after a few generations of growth. Since this stress appears to inhibit complete septation during bacterial cell division, it is sufficient to permit the formation of bacterial filaments. This data also explains why filamentation is not achieved at higher salt concentration: the stress is toxic, inhibiting bacterial growth and cell division, and causing cell death.

### Example 3 - Use of biologically compatible density gradient media to separate intact minicells from parent bacteria and other contaminants.

Post-differential centrifugation of the bacterial cell / minicell culture, a significant number of bacterial cell contaminants were eliminated by density gradient centrifugation using a biologically compatible medium. OptiPrep™ (Axis-Shield PLC, Dundee, Scotland), which is a sterile 60% (w/v) solution of iodixanol (5,5'-[(2-hydroxy-1-3 propanediyl)-bis(acetylamino)] bis [N,N'-bis(2,3dihydroxypropyl-2,4,6-triiodo-1,3-benzenecarboxamide])in water, constitutes a biologically compatible medium. 6% to 12% gradients were prepared in 25ml polypropylene clear centrifuge tubes (Livingstone International Pty Ltd, Sydney, Australia) and 1ml of the minicell/bacterial cell suspension was layered on each gradient. The tubes were centrifuged at 2,000g / 20min. Twenty-three 1ml samples were collected from the tube and analyzed by light microscopy using a 100x magnification oil-immersion objective. The results showed 3 main segments as shown in Figure 5A. The top segment contained largely minicells with contaminating bacterial cells and bacterial blebs. A second lower segment carried mainly bacterial cells but they appeared approximately 2 to 3 times longer than a minicell. The pellet carried mainly bacterial cells.

The crude minicell suspension (upper band; **Fig. 5A**) was collected and centrifuged at 13,200 rpm / 30min in Eppendorf tubes. The pellet was resuspended in 2ml of sterile BSG and re-processed on OptiPrep™ gradients as above. The results showed (Fig. 5B) that there was a relatively smaller bacterial pellet, the middle band was a lot clearer and the minicell band appeared distinct. All zones (2ml volumes) in the tube were analysed by light microscopy as above and the results were similar except that the minicell band appeared to carry very few bacterial contaminants. The minicell band was collected as before and re-processed through a third OptiPrep™ gradient as above. This resulted in a clear band (Fig. 5C) and when analyzed microscopically, it revealed the presence of mainly minicells with very few bacterial contaminants. The experiments were repeated 10 times with similar results.

### Example 4 - Significant reduction of free endotoxin from purified intact minicell preparations.

The endotoxins of Gram-negative bacteria are lipopolysaccharide (LPS) molecules with three distinct domains referred to as lipid A, core oligosaccharide and O-polysaccharide. The lipid A and core oligosaccharide comprise the endotoxin core and are relatively conserved among different Gram-negative bacterial species. Lipid A is the toxic moiety of endotoxin, and is covalently linked to core oligosaccharide in all LPS (Reitschel *et al.,* 1991).

The residual free endotoxin was removed from purified minicell preparations as follows. Magnetic beads coated with Protein G (Dynal Biotech, Oslo, Norway) were conjugated to goat anti-Lipid A polyclonal antibody (Biodesign, Saco, Maine, USA). This antibody is cross-reactive with a range of different Gram-negative bacterial LPS species including *S*. *typhimurium* LPS. The conjugation reaction was carried out by incubating (O/N with gentle mixing at 4°C) Dynabeads-Protein G that were washed 3 times in 0.5ml 0.1M Na-Phosphate Buffer (pH 5.0) with the anti-LipidA antibody. The excess antibody was eliminated by washing the Dynabeads-Protein G / anti-LipidA conjugate three times with 0.5ml 0.1M Na-Phosphate Buffer (pH 5.0). The conjugate was resuspended in 300ul of the same buffer and 50ul was used to treat 500ul of the purified minicell suspension for free endotoxin removal. The co-incubation was carried out for 1hr at 4°C followed by placing the tube on a magnet (Dynal) and collecting the minicell supernatant. The treatment was carried out 3 times with fresh Dynabeads-Protein G / anti-LipidA conjugate to ensure maximal removal of residual free endotoxin.

### Example 5 - Enumeration of intact minicell, bacterial cell and endotoxin counts throughout the minicell purification procedure.

This experiment was designed to determine the kinetics of the minicell purification procedure with respect to minicell yield, reduction in parent bacterial counts and reduction of free endotoxin. The complete purification procedure was carried out three times and samples were collected at each of 12 steps outlined below. Each sample was analyzed for minicell and bacterial cell counts by Flow Cytometry and viablility counting on agar plates: Endotoxin Units (EU) were also determined for each sample by the LAL assay (Charles River Laboratories, Inc. Wilmington, MA, USA), carried out by Australian Microbiology Services Pty Ltd (Sydney, Australia). The complete purification process is briefly outlined below.

Recombinant *S*. *typhimurium min*CDE- strain carrying a high-copy number plasmid (nucleic acid marker) was grown overnight (O/N) at 37°C / shaking in 25ml Trypticase Soy Broth (TSB). Six flasks each containing 1L of TSB were then inoculated with 2ml of the O/N culture and incubated further at 37°C / shaking (**Sample 1**). The culture was differentially centrifuged at 2000g / 20min in a benchtop centrifuge to sediment a significant number of bacterial cells. The supernatant (**Sample 2**) was collected and concentrated by passing it through a 0.1µm cross-flow filter. The suspension was further concentrated by centrifugation at 13,200rpm / 60min in an Eppendorf centrifuge and the minicell / bacterial cell pellet was resuspended in 16ml of sterile BSG (**Sample 3**). Sixteen density gradients (6 % to 12 %) were prepared in 25ml polypropylene clear centrifuge tubes (Livingstone International Pty Ltd, Sydney, Australia) using a biologically compatible medium OptiPrep™ (Axis-Shield PLC, Dundee, Scotland), and 1ml of the minicell/bacterial cell suspension was layered on each gradient. The tubes were centrifuged at 2,000g / 20min and the minicell band (**Fig. 5A**) was collected from the top of each tube with a syringe. Approximately 24ml of the crude minicell suspension (**Sample 4**) was collected and centrifuged at 13,200 rpm / 30min in Eppendorf tubes. The pellet was resuspended in 12ml of sterile BSG and re-processed on 12 OptiPrep™ gradients as above. The minicell bands (Fig. 5B) were collected as before (**Sample 5**) and re-processed through 4 OptiPrep™ gradients as above. This resulted in a clear diffuse minicell band (Fig. 5C) in each tube and was collected (**Sample 6**). The minicell suspension was added to 1L of TSB and incubated for 2hr / 37°C / standing to reactivate contaminating bacterial cells (**Sample 7**). NaCl (final concentration of 5% w/v) was added to the suspension to stress the living bacterial cells and inhibit the process of septum formation during cell division. The suspension was incubated for 2hrs 37°C / standing to ensure that most of the contaminating bacterial cells are converted into bacterial filaments (**Sample 8**). Broad spectrum antibiotics, Gentamycin (200ug/ml) and Kanamycin (200ug/ml) were added to the suspension and incubated O/N at 37°C to kill all living bacterial cells (**Sample 9**). The suspension was passed through a 0.2µm cross-flow filter for buffer exchange and the suspension was reconstituted in sterile BSG (**Sample 10**). This process eliminated all contaminants smaller than 0.2µm for example free endotoxin, lysed bacterial and minicell fragments, nucleic acids and TSB nutrients. Bacterial filaments from the minicell suspension were then eliminated by passing the solution through a 0.45µm Dead-end filter. The suspension was concentrated to a 50ml volume by passing it through a 100kDa cross-flow filter and then centrifuged at 13,200 rpm/20min to pellet the minicells. The supernatant was discarded and the pellet was resuspended in 1ml sterile BSG (Sample 11). The residual free endotoxin was removed using Dynabeads-Protein G conjugated to anti-lipidA antibody as described in Example 4. This resulted in **Sample 12.**

Enumeration of minicells and bacterial cells was carried out by Flow Cytometry as follows. Each sample from the minicell purification procedure was diluted appropriately to carry approximately 10⁸ to 10¹⁰ minicells and 250µl of the sample was incubated with 3.3µM Syto9 green fluorescent dye (Molecular Probes, Eugene, OR, USA). This dye permeates intact and damaged membranes of bacterial cells and binds to endogenous nucleic acids resulting in green fluorescent bacterial cells. In our preliminary studies we had shown that it also permeates the minicell membrane and results in green fluorescent recombinant minicells. All samples were counted on the FACSCalibur flow cytometer (Becton Dickinson, San Jose, CA, USA) using the Cellquest acquisition and analysis software (Becton Dickinson). Counts were performed over 30 seconds on Medium Flow Rate with a 37V threshold on a green fluorescence "FL1" trigger detecting between 515-545 nm. FL1 PMT voltage was set to 550V. Side scatter "SSC" PMT voltage was set to 524V. Appropriate sample dilution returned between 5000-50000 particles per 30sec and final particle count was an average of 5 repetitions. Minicells and Bacteria were distinguished based on the basis of their differences in SSC and Green Fluorescence.

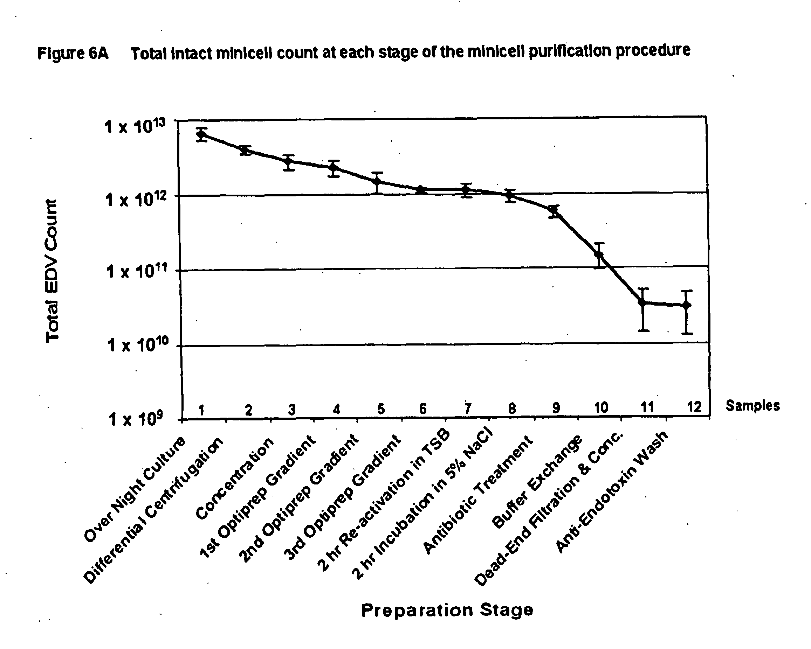

The results showed (Figs 6A-C) that at the start of the purification process, the 6L bacterial/minicell culture carried approximately 5 x 10¹² minicells (Fig 6A) and gradual losses occurred through the purification procedure to result in an yield of approximately 5 x 10¹⁰ minicells at the end.

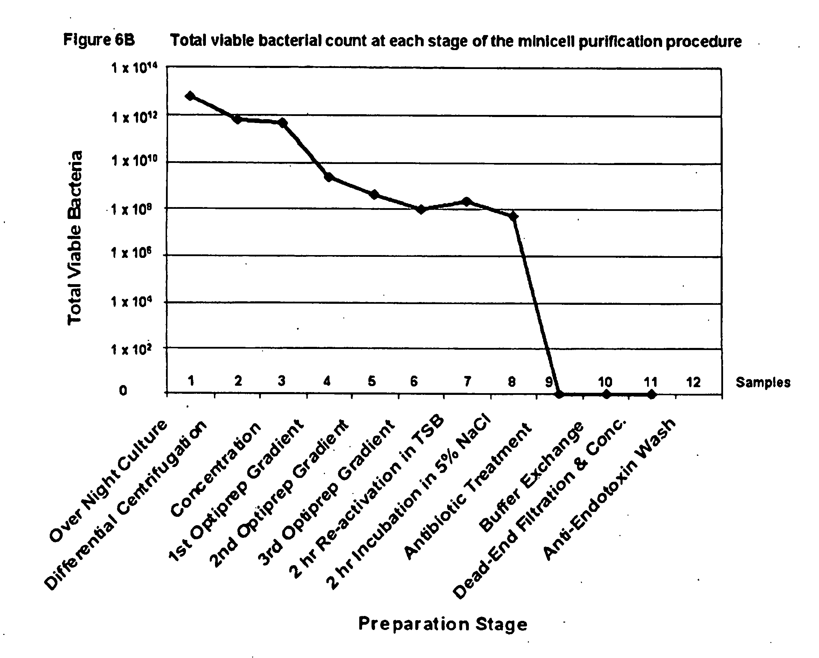

The parent bacterial cell count (Fig. 6B) at the commencement of the procedure was similar to the minicell numbers, *i.e*., approximately 5 x 10¹² bacteria. The purification process resulted in the elimination of over 1000-fold bacterial cells at the point of antibiotic treatment. At this point the ratio of minicell:bacterial cell was over 100:1 and hence Flow cytometry measurements could not detect bacterial cells in the samples analyzed. After the antibiotic treatment, all samples analyzed by viable plate count assay revealed the presence of no live bacterial cells. Quantitative PCR analysis for the presence of genomic DNA in the final purified sample revealed the presence of less than 1 parent bacterial cell (dead cell) in 10¹¹ purified minicells.

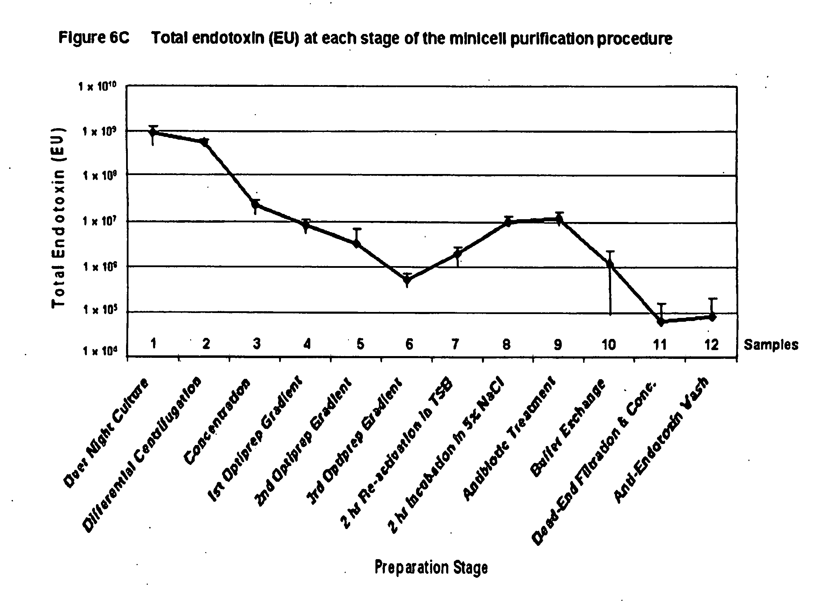

The free endotoxin count in each sample (Fig. 6C) showed that at the start of the purification process the sample carried approximately 16⁹ Endotoxin Units (EU). The EU decreased to approximately 10⁶ after the third gradient purification step (Fig. 6C; Sample 6). The EU increased again once the minicell/residual bacterial cell suspension was incubated in TSB for salt-induced filamentation (Fig. 6C; Samples 7-9) but was reduced to 10⁴ to 10⁵ EU in subsequent purification steps.

It is known that in aqueous solutions, purified LPS can be found as free LPS and as micelles since the Lipid A moiety is hydrophobic, while the carbohydrate segment (core polysaccharide and O-polysaccharide) are hydrophilic. This results in micelles where the hydrophobic Lipid A is buried while the hydrophilic polycaccharide interacts with the aqueous environment. These micelles are known to be present in a range of different molecular sizes and can be as large as several million Daltons. The LPS attached to a bacterial cell or a minicell has the Lipid A portion buried within the bilayer membrane and is not an endotoxin. Free LPS is an endotoxin since the Lipid A moiety is available to interact with mammalian cell membranes resulting in serious endotoxic effect. It is not known whether LPS micelles are endotoxins *in-vivo.*

The minicells of this invention are useful for in-vivo therapeutic applications and hence the purification procedure is focused on the removal of free LPS, *i.e.,* endotoxin. The LAL assay, however, measures all three forms of LPS: free form (endotoxin), micellar form (studies below suggest that this is not endotoxin) and minicell-surface bound (not endotoxin). In contrast the anti-Lipid A antibody only appears to bind to and eliminate the free LPS since the antigen-binding sites of the antibody cannot access Lipid A in micelles or in intact minicells since the antigen-binding sites are buried.

### Example 6 - Determination of free endotoxin levels in purified minicell preparations.

To demonstrate the above, a series of further experiments were conducted to determine what form of LPS is actually measured by the LAL assay. The purified minicell preparation was sequentially treated 5 times for free LPS removal using freshly prepared Dynabeads-Protein G / anti-Lipid A conjugate and from each purification the following samples were analyzed by the LAL assay; (a) purified minicell suspension that is expected to carry all 3 forms of LPS, *i.e.,* free form, micellar and minicell-surface membrane bound, (b) the recovered Dynabeads-Protein G / anti-Lipid A conjugate which presumably carries bound free form LPS, (c) the supernatant following centrifugation of minicell suspension at 13,200 rpm / 20min after Dynabeads-Protein G / anti-Lipid A treatment. This presumably carries the micellar form LPS since the free form should be removed by anti-Lipid A and the minicell-bound LPS should be found in the minicell pellet, (d) the pellet from (c) that was resuspended in sterile pyrogen-free BSG. A series of controls were also included, *e.g*., purified *S. typhimurium* LPS (Sigma Chemical Company, St. Louis, MO, USA) treated with Dynabeads-Protein G / anti-Lipid A conjugate to confirm that the anti-Lipid A antibody did bind to and eliminate free form LPS, diluent samples to ensure that diluents did not contribute to the observed EU, *etc.*

The results showed (Table 1, below) that as expected, most of the EU that is measured by the LAL assay is associated with the minicell-surface membrane bound LPS (column D) which is not an endotoxin as seen in the in-vivo studies in example 7. This result is similar to that seen in Fig. 6C (Sample 12). The supernatant (column C) also carried a significant amount of EU and presumably that is the micellar form of LPS since each sample had previously been processed with the Dynabeads-Protein G / anti-Lipid A conjugate. The interesting result was seen in column B which showed that each time the anti-Lipid A treatment is carried out, the only amounts of free form LPS that could be found in the purified minicell preparation was between 20EU to 45EU. This value is much lower than the current endotoxin standards for lot release of parenteral pharmaceuticals which is 350EU / dose (Grandics, 2000).

**Table 1**

| | A | B | C | D |
|---|---|---|---|---|
| Sample | Minicell suspension | Recovered Dynabeads-Protein G/anti-Lipid A | Supernatant after minicells are pelleted by centrifugation | Minicell pellet resuspended |
| | EU/ml of original solution | | | |
| After 1° free endotoxin removal process | 15,560 | 43.8 | 3,490 | 9,910 |
| After 2^{nd} free endotoxin removal process | 18,910 | 35.1 | 2,250 | 16,940 |
| After 3^{rd} free endotoxin removal process | 26,920 | 19.0 | 2,143 | 10,090 |
| After 4^{th} free endotoxin removal process | 20,230 | 25.2 | 1,870 | 11,620 |
| After 5^{th} free endotoxin removal process | 15,400 | 27.8 | 1,361 | 15,080 |

### Example 7 - In-vivo confirmation that purified minicells carry insignificant levels of endotoxin.

Purified minicell preparations were sequentially (3 times) treated for endotoxin removal using the Dynabeads-Protein G / anti-Lipid A conjugate procedure described above. Each of the preparations carrying 16⁹ purified minicells, *i.e*., pre-endotoxin removal, and after first-, second- and third- endotoxin removal steps, was injected into the tail vein of 6 week old female athymic nude mice (5 mice per group). The mice used in this example were purchased from Animal Resources Centre, Perth, WA, Australia, and all animal experiments were performed in compliance with the guide of care and use of laboratory animals and with Animal Ethics Committee approval. The experiments were performed in the NSW Agriculture accredited small animal facility at EnGeneIC Pty Ltd (Sydney, NSW, Australia). The mice were carefully observed over a period of 4 weeks to record any signs of endotoxin shock, *e.g*., fever, lethargy, loss of appetite and weight, and subsequent death. The results showed that without the endotoxin removal procedure most mice rapidly developed fever and were lethargic within the first 12 hrs. Most of the animals died within two weeks. The mice receiving minicells after the first endotoxin removal procedure were more stable and exhibited a mild fever for the first 24hrs. However, the mice recovered after 3 days. Mice receiving purified minicells that had undergone two and three rounds of endotoxin removal showed no adverse side effects and remained healthy. This suggested that the novel step of free-endotoxin removal was essential if the minicells are to be utilized for pharmaceutical purposes within a mammalian host.

### CITED PUBLICATIONS

Brahmbhatt, "Cloning and molecular characterization of the rfb gene cluster of Salmonella ryphimurium," Ph.D. Thesis, University of Adelaide, Australia (1987).
Britton et al., "Characterization of a prokaryotic SMC protein involved in chromosome partitioning," Genes Dev. 12: 1254 (1998).
Clark-Curtiss & Curtiss, "Analysis of recombinant DNA using Escherichia coli minicells," Methods Enzymol. 101: 347 (1983).
de Boer et al., "Roles of MinC and MinD in the site-specific septation block mediated by the MinCDE system of Escherichia coli," J. Bacteriol. 174: 63 (1992).
Dorward et al., "Export and intercellular transfer of DNA via membrane blebs of Neisseria gonorrhoeae," J. Bacteriol. 171: 2499 (1989).
Forbes, "Crossflow microfiltration," Australian J. Biotechnology 1: 30 (1987).
Grandics, P. Pyrogens in parenteral pharmaceuticals. Pharmaceutical Technology. (2000).
Frazer & Curtiss, "Production, properties and utility of bacterial minicells," Curr Top Microbiol. Immunol. 69: 1 (1975).
Harlow et al., "Cloning and characterization of the gsk gene encoding guanosine kinase of Escherichia coli, " J. Bacteriol. 177: 2236 (1995).
Harry, "Bacterial cell division: Regulating Z-ring formation," Mol. Microbiol. 40: 795 (2001).
Hiraga et al., "Chromosome partitioning in Escherichia coli: novel mutants producing anucleate cells," J. Bacteriol. 171: 1496 (1989).
Hu & Lutkenhaus, "Topological regulation of cell division in Escherichia coli involves rapid pole to pole oscillation of the division inhibitor MinC under the control of MinD and MinE," Mol. Microbiol. 34: 82 (1999).
Ireton et al., "spo0J is required for normal chromosome segregation as well as the initiation of sporulation in Bacillus subtilis," J. Bacteriol. 176: 5320 (1994).
Katsui et al., "Heat-induced blebbing and vesiculation of the outer membrane of Escherichia coli, " J. Bacteriol. 151: 1523 (1982).
Kihara et al., "Analysis of a FliM-FliN flagellar switch fusion mutant of Salmonella typhimurium," J. Bacteriol. 178: 4582 (1996).
Levin et al., "Identification of Bacillus subtilis genes for septum placement and shape determination," J. Bacteriol. 174: 6717 (1992).
Matsuzaki et al., "Interactions of an antimicrobial peptide, magainin 2, with outer and inner membranes of Gram-negative bacteria," Biochim Biophys. Acta. 1327: 119 (1997).
Okada et al., "Possible function of the cytoplasmic axial filaments in chromosomal segregation and cellular division of Escherichia coli," Sci. Prog. 77: 253 (1993-94).
Okada et al., "Cytoplasmic axial filaments in Escherichia coli cells: possible function in the mechanism of chromosome segregation and cell division," J. Bacteriol. 176: 917 (1994).
PCT/IB02/04632. Intact minicells as vectors for DNA transfer and gene therapy in vitro and in vivo.
Raskin & de Boer, "MinDE-dependent pole-to-pole oscillation of division inhibitor MinC in Escherichia coli, "J. Bacteriol. 181: 6419 (1999).
Reitschel ET, Seydel U, Zähringer FU et al. Bacterial endotoxin: molecular relationships between structure and activity. Infect Dis Clin N Am. 5: 753 (1991).
Reeve, "Use of minicells for bacteriophage-directed polypeptide synthesis," Methods Enzymol. 68: 493 (1979).
Reeve & Cornett, "Bacteriophage SPO1-induced macromolecular synthesis in minicells of Bacillus subtilis," J. Virol. 15: 1308 (1975).
Sancar et al., "Simple method for identification of plasmid-coded proteins," J. Bacteriol. 137: 692 (1979).
Stewart & D'Ari, "Genetic and morphological characterization of an Escherichia coli chromosome segregation mutant," J. Bacteriol. 174: 4513 (1992).
Wachi et al., "New mre genes mreC and mreD, responsible for formation of the rod shape of Escherichia coli cells," J. Bacteriol. 171: 6511 (1989).

## Claims

1. A purified bacterially derived minicell preparation containing fewer than about 1 contaminating parent bacterial cell per 10¹⁰ minicells.

2. The preparation of claim 1, wherein said preparation contains fewer than about 1 contaminating parent bacterial cell per 10¹¹ minicells.

3. The preparation of claim 1 or 2, wherein said preparation is substantially free of endotoxins.

4. The preparation of any one of claims 1 to 3, wherein said preparation is substantially free of cellular debris.

## Patentansprüche

1. Aufgereinigtes, bakteriell abgeleitetes Minizellenpräparat mit weniger als etwa 1 kontaminierenden bakteriellen Mutterzelle pro 10¹⁰ Minizellen.

2. Präparat nach Anspruch 1, wobei das Präparat weniger als etwa 1 kontaminierende bakterielle Mutterzelle pro 10¹¹ Minizellen enthält.

3. Präparat nach einem der Ansprüche 1 bis 2, wobei das Präparat im Wesentlichen frei von Endotoxinen ist.

4. Präparat nach einem der Ansprüche 1 bis 3, wobei das Präparat im Wesentlichen frei von Zelltrümmern ist.

## Revendications

1. Préparation de minicellules purifiée bactériellement derivée contenant moins d'environ 1 cellule bactérienne mère contaminante pour 10¹⁰ minicellules.

2. La préparation de la revendication 1, dans laquelle ladite préparation contient moins d'environ 1 cellule bactérienne mère contaminante pour 10¹¹ minicellules.

3. La préparation de la revendication 1 ou 2, dans laquelle ladite préparation est essentiellement exempte d'endotoxines.

4. La préparation de l'une quelconque des revendications 1 à 3, dans laquelle ladite préparation est essentiellement exempte de débris cellulaires.
